Europäisches Patentamt

European Patent Office

Office européen des brevets

(11) Publication number: **0 147 145**
**A2**

# (12) EUROPEAN PATENT APPLICATION

(21) Application number: **84308758.6**

(22) Date of filing: **14.12.84**

(51) Int. Cl.⁴: **C 12 N 15/00**
**C 12 N 1/20**
**//(C12N1/20, C12R1:465)**

(30) Priority: **16.12.83 GB 8333544**

(43) Date of publication of application:
**03.07.85 Bulletin 85/27**

(84) Designated Contracting States:
**AT BE CH DE FR GB IT LI LU NL SE**

(71) Applicant: **Pfizer Limited**
**Ramsgate Road**
**Sandwich Kent CT13 9NJ(GB)**

(84) Designated Contracting States:
**GB**

(71) Applicant: **Pfizer Corporation**
**Calle 15 1/2 Avenida Santa Isabel**
**Colon(PA)**

(84) Designated Contracting States:
**BE CH DE FR IT LI LU NL SE AT**

(72) Inventor: **Hunter, Iain Stark, Dr.**
**6 Lammermuir Gardens Baljaffrey**
**Bearsden Glasgow G61(GB)**

(72) Inventor: **Friend, Eric John**
**Clery Mill Lane**
**Worth Sandwich Kent(GB)**

(74) Representative: **Graham, Philip Colin Christison et al,**
**Pfizer Limited Ramsgate Road**
**Sandwich, Kent CT13 9NJ(GB)**

(54) **Streptomycete host cells of reduced restriction activity.**

(57) Streptomycete host cells having reduced restriction activity are obtained by (a) mutagenising a strain of a Streptomycete species to provide host cells of differing genetic characteristics; (b) growing the surviving host cells and forming protoplasts thereof; (c) transforming the protoplasts with plasmid DNA from another source, such plasmid DNA containing an origin of replication and a selective marker for the Streptomycete host, as well as any DNA sequences necessary for replication and selective culture from the other source; (d) regenerating host cells from the transformed protoplasts containing the plasmid DNA; (e) selecting those host cells which have received the plasmid DNA, and been unable to restrict it, by selecting for expression of the selective marker; (f) confirming that the host cells have reduced restriction activity; and (g) if desired, repeating steps (a) to (f) to reduce further the restriction activity of the host cells and confirm their further reduced restriction activity. Such cells are more able to accept recombinant DNA from another source and are therefore of value for transforming into forms for expression of industrially useful products.

- 2 -

The invention relates to Streptomycete host cells having reduced restriction activity and methods for obtaining such cells. It is particularly concerned with a method for obtaining host cells of Streptomycete species which, by virtue of their reduced restriction activity, are more able to accept recombinant DNA from another source and are therefore of particular value for transformation by such DNA into forms in which expression of useful industrial products will take place.

The cells of most naturally occurring strains of Streptomycete species contain restriction enzymes, i.e. enzymes which recognise and cleave susceptible sites in foreign DNA (even DNA from other Streptomycete species) entering the cell and therefore, either alone or in association with modification systems, restrict the ability of such DNA to survive and replicate in the cell. However, mutation of these strains can produce strains in which the cells are lacking in these restriction enzymes and are therefore more able to accept foreign DNA entering the cell and allow it to replicate. The problem is to find and select such 'restrictionless' strains.

One method of finding and selecting strains of Streptomycete species whose cells are lacking in restriction enzymes is to expose mutagenised strains to a bacterial virus (or bacteriophage) and then to select those strains which are susceptible to infection by the virus. Such strains may then be found to be more able to accept recombinant DNA from other sources. Such a method has been described by Chater and Wilde in the Journal of General Microbiology, 116, 323-334 (1980). This method has the disadvantage, however, that bacterial viruses are highly specific and very few will replicate in and thereby infect Streptomycete species. Moreover, bacterial viruses are hazardous to use and their use for selection purposes would carry the risk of cross-infection of industrially important strains used for the production of antibiotics and other products.

According to the present invention, a method for obtaining Streptomycete host cells having reduced restriction activity comprises the steps of:

(a) mutagenising a strain of a Streptomycete species to provide host cells of differing genetic characteristics;

(b) growing the surviving host cells and forming protoplasts thereof;

(c) transforming the protoplasts with plasmid DNA from another source, such plasmid DNA containing an origin of replication and a selective marker for the Streptomycete host, as well as any DNA sequences necessary for replication and selective culture from the other source;

(d) regenerating host cells from the transformed protoplasts containing the plasmid DNA;

(e) selecting those host cells which have received the plasmid DNA, and been unable to restrict it, by selecting for expression of the selective marker;

(f) confirming that the host cells have reduced restriction activity; and

(g) if desired, repeating steps (a) to (f) to reduce further the restriction activity of the host cells and confirm their further reduced restriction activity.

Also according to the invention, a method for obtaining Streptomycete host cells having reduced restriction activity comprises steps (a) to (f) and, if desired, step (g), as already defined, wherein in step (f) the selected host cells from step (e) are further selected for loss of the plasmid DNA from another source introduced in step (c) and tested for reduced restriction activity, thereby providing host cells of reduced restriction activity without the selective marker.

Also according to the invention, there are provided Streptomycete host cells having reduced restriction activity produced by the method of the invention.

The use of plasmid DNA to select host cells of reduced restriction activity has the advantage that plasmid DNA, especially DNA from bacterial plasmids, has a much wider host range, i.e. ability to replicate in host cells, than bacterial viruses, and, being formed by genetic engineering techniques, can be assured to be non-hazardous. The invention may be adapted to provide a simple, convenient and inexpensive method for obtaining host cells of any Streptomycete species altered to reduce its restriction activity.

The invention makes available a system that provides a means for selecting host cells (hereinafter called host 1) that are deficient in restriction activity by using plasmid DNA which has been grown up in another host (hereinafter called host 2). Normally, when DNA is grown up in this host (host 2) and used to transform host 1, no transformants are obtained, or at best very few transformants are obtained. This is because host 1 contains one or more enzymes (restriction enzymes) which digest (restrict) the DNA from host 2. In many recombinant DNA experiments involving Streptomycetes, the protocols demand that DNA is transferred (transformed) from one host to another. The presence of restriction enzymes within the recipient host often makes this operation impossible or at best very inefficient. The ability to carry out the transfer (transformation) of DNA between Streptomycete strains and species at high efficiency is crucial to the performance of present day methods in Streptomycete recombinant DNA research.

Only one effective method has been described for obtaining restriction-deficient strains of Streptomyces (Chater and Wilde, loc. cit.) and that method can have serious limitations. It involves using a non-pathogenic bacterial virus, which can be recognised to have infected a Streptomycete by forming plaques within a lawn of the Streptomycete growing on an agar plate. Under certain circumstances it is possible to detect restriction-minus mutant cells of Streptomycetes by the plaques formed after infection with the virus. There are two major disadvantages to this approach. Firstly, both hosts (host 1 and host 2) have to be susceptible to infection by the virus. Few Streptomyces can be infected by the virus and indeed many Streptomycete species may have evolved to become immune to infection by the virus. Secondly, the use of any bacterial virus constitutes a risk to other Streptomycete strains being used in the same laboratory or in a laboratory nearby. Whereas all manipulations with the virus would be carried out using good microbiological practice with the risk of cross-infection minimal, there is still a small but finite risk attached to the use of the virus in the laboratory. (This risk is only associated with infection of other Streptomyces and not with the infection of the operators. The virus used would be non-pathogenic to humans.) The problem of cross-infection of Streptomycete strains is a real one in the industrial context since Streptomycete organisms used for commercial operations at large scale could be infected by the virus while being used with another Streptomycete organism in an adjacent laboratory.

Alternative methods for obtaining Streptomycete host cells which have reduced restriction activity are therefore needed. Transfer of a DNA plasmid into a host cell can be recognised if the plasmid contains a selective marker. The present invention embodies this principle to recognise when a mutant Streptomycete cell has arisen which is altered in its ability to restrict DNA, which has been grown up in another host.

For the purposes of the present invention, a DNA plasmid is defined herein as any agent, not limited to recombinant plasmids, but excluding bacteriophages and viruses, consisting of a DNA molecule to which one or more additional DNA segments may have been added.

A marker is defined herein as a gene, or a combination of genes, of known function in a chromosome or a recombinant DNA cloning vector.

As already described, the present invention can be used to obtain Streptomycete host cells which have reduced restriction activity. Without an effective system to obtain and select for these mutant strains of Streptomyces with reduced restriction activity, the application of recombinant DNA techniques to Streptomyces and particularly to interspecific DNA manipulations within the Streptomyces is severely limited.

The present invention is particularly versatile since it can be applied to any Streptomycete able to, or thought to be able to, act as a host for a plasmid. Since some plasmids are able to propagate themselves in diverse species of Streptomyces, and since a large number of plasmids able to propagate in Streptomyces are known the invention is potentially of wide application.

The individual steps of the invention will now be described in more detail.

In step (a) the Streptomycete strain of host cells may be allowed to undergo spontaneous mutagenesis, but preferably mutagenesis is accelerated by any of the various well known techniques.  For example, the strain may be exposed to high energy radiation, such as ultra violet light, e.g. 254nm, or 356nm in the presence of 8-methoxypsoralen, or treated with a chemical mutagen such as N-methyl-N$^1$-nitrosoguanidine or an alkylating agent.  The preferred mutagen is N-methyl-N$^1$-nitrosoguanidine.

In step (b) the host cells surviving after mutagenesis are grown by culturing on a conventional medium, e.g. tryptone-soya broth or a yeast extract/malt extract medium (preferably containing an agent such as glycine to make the cells more susceptible to protoplast formation by modifying the cell walls), separated by centrifuging and re-suspended in a buffered aqueous sucrose solution.  Protoplasts of the cells are then formed by treatment with known agents which disrupt the cell walls, e.g. lysozyme and/or achromopeptidase.

In step (c) plasmid DNA from the other source is used to transform the protoplasts, by mixing a buffered aqueous suspension of the plasmid DNA with the protoplast suspension and adding an agent which promotes transfer of plasmid DNA into the protoplasts, e.g. a 20 to 25% (w/v) aqueous solution of polyethylene glycol of molecular weight 1000 to 6000.  Transformation of the protoplasts with the plasmid DNA is usually complete in about 1 minute from adding the promoting agent.

In step (d) the cell walls of the host cells are regenerated by first removing the promoting agent from effective contact with the protoplasts, e.g. by centrifuging the mixture to separate the protoplasts and re-suspending the protoplasts in buffered aqueous sucrose solution, or by diluting the mixture with buffered aqueous sucrose solution to reduce the concentration of the promoting agent to an ineffective level, e.g. a 5 to 10 fold dilution.

Aliquots of the re-suspended or diluted protoplast mixture are then spread on agar plates, using a medium containing sucrose, mineral salts and a rich source of protein, e.g. yeast extract.

In step (e), host cells which have been transformed with plasmid DNA from the other source, and have not restricted it, are selected for expression of the marker. In the case where the marker is one for resistance to an antibiotic, the host cells are exposed to the antibiotic and those which continue to grow are selected. This may be done either by spreading a thin layer of liquid or soft agar medium containing the antibiotic over all the growing colonies, in which case only colonies of resistant cells will continue to grow, or replicating individual colonies on separate agar plates containing the antibiotic to find those which are resistant and therefore continue to grow.

Finally, in step (f), confirmation that the selected host cells provided by step (e) do in fact possess reduced restriction activity with respect to foreign DNA, and have not merely modified the plasmid DNA to a form which is not restricted by the host cells, may be effected by obtaining from them cells which have lost the plasmid DNA and testing them for restriction activity by reintroducing foreign DNA by protoplast formation and transformation using the techniques already described in steps (c), (d) and (e). Cells which have lost the plasmid DNA are obtained from the selected host cells by growing colonies of them, in the presence and absence of the same antibiotic as before, and selecting those which are no longer resistant to the antibiotic. Loss of plasmid DNA can, if desired, be accelerated by forming protoplasts of the selected host cells and regenerating the cells before growing the colonies in the presence and absence of the antibiotic.

As already mentioned, steps (a) to (e) of the process of the invention can be repeated, if necessary, if the procedure of step (f) shows that the restriction activity of the host cells transformed with the plasmid DNA has not been reduced to the desired extent.

Plasmid DNA for use in the step (c) of the process of the invention may be derived from any plasmid, provided that it contains an origin of replication for the Streptomycete host and a selective marker which can be recognised in that host. Thus, it may be derived from a plasmid from another Strepomycete strain, already containing an origin of replication for the Streptomycete host, by adding to it (by well-known genetic engineering techniques) a selective marker for resistance to an antibiotic, e.g. thiostrepton, neomycin or viomycin. Alternatively, it may be derived from a plasmid from a micro-organism other than a Streptomycete, e.g. a bacterium such as <u>Escherichia coli</u>, or a yeast, by adding to it (by similar techniques) a fragment of Streptomycete DNA containing an origin of replication for Streptomycetes, as well as the selective marker.

Preferably the plasmid DNA is of sufficient size to provide a number of sites in its structure which are susceptible to attack by the restriction enzymes of the host cell before mutagenesis. If the plasmid DNA contains only one such site, the chances of it being modified by the host cell to a form in which it is not restricted are relatively high, and the number of colonies which are selected in step (e), but which do not possess reduced restriction activity, will also be relatively large, and the number having reduced restriction activity will be correspondingly small.

Suitable plasmids, from which plasmid DNA for use in the invention can be derived, include pIJ101, present in Streptomyces lividans ISP5434 (DSM40434) the isolation and characterisation of which is described by Kieser et al in Mol. Gen. Genet. 185, 223-238 (1982) and which is obtainable from the John Innes Institute, Colney Lane, Norwich, England. Another suitable plasmid of very similar structure is pS10147, present in Streptomyces coelicolor ATCC10147, as described by Pernodet and Guerinedu in Mol. Gen. Genet. 182, 53-59 (1981). Other suitable plasmids, available from strains of Streptomycetes containing them which are deposited in public culture collections, include those given in Table I.

- 12 -

TABLE I

| Plasmid | Deposited micro-organism* containing it | |
|---------|----------------|---|
| SLP 1.1 | S.lividans | NCIB 11417 |
| SLP 1.2 | " | NCIB 11499 |
| SLP 1.3 | " | NCIB 11500 |
| SLP 1.4 | " | NCIB 11501 |
| SLP 1.5 | " | NCIB 11502 |
| SLP 1.6 | " | NCIB 11503 |
| pUC 1 | S.fradiae | NRRL 11443 |
| pUC 2 | " | NRRL 12323 |
| pUC 3 | S.sp.3022a | NRRL 11441 |
| pUC 6 | S.espinosa 23724a | NRRL 11439 |
| pUC 7 | S.espinosa acanthus | NRRL 11081 |
| pUC 8 | S.fradiae | NRRL 11445 |
| pUC 9 | " | NRRL 11440 |
| pSF 588 | S.albofaciens | FERM-P5267(BP122) |
| pSF 619 | S.hygroscopicus | FERM-P5269(BP123) |
| pSF 689 | S.platensis | FERM-P5002(BP121) |
| pSF 765 | S.fradiae | FERM-P5270(BP124) |
| pSF 701-1 | S.griseochromogens | FERM-P5000(BP120) |
| pSVH 1 | S.venezuelae | DSM 40755 |

* S = Streptomyces

- 13 -

Suitable plasmids, available from micro-organisms other than Streptomycetes and from which plasmid DNA for use in the invention can be derived, include those given in Table II.

TABLE II

| Plasmid | Deposited micro-organism* containing it | |
|---------|-----------------------------------------|---|
| pBR 322 | E.coli RR1 (pBR322) | ATCC 31344 |
| pOP 203 | E.coli HB101 (pOP203) | NRRL B-11355 |
| pUB 110 | B.subtilis (pUB110) | NCIB 11624 |
| pAB 124 | B.subtilis (pAB124) | NCIB 11622 |
| pCGS 28 | E.coli (CGE24) | ATCC 31929 |
| pCGE 21 | S.cerevisiae (CGY116) | ATCC 20623 |

\* E = Escherichia
  B = Bacillus
  S = Saccharomyces

The particular plasmid, from which plasmid DNA for use in one Example of the present invention which follows has been derived, is pIJ 101. This plasmid, of size about 8.9 kb (1 kb = 1000 base pairs of nucleotides in the double-stranded DNA molecule), has been modified by well-known genetic engineering techniques, first by addition of a thiostrepton-resistant marker in order to yield the plasmid pIJ 303 (c. 10.8 kb) and then by in vivo deletion of DNA incompatible with Streptomyces rimosus to yield the plasmid pPZ 12 (c. 6.2 kb). The plasmid is then ready for use in transforming protoplasts of mutagenised S.rimosus host cells in step (c) of the process of the invention. A strain of Streptomyces albus, designated AT 95, containing the plasmid pPZ 12 was deposited for purposes of patent procedure under the Budapest Treaty in the Culture Collection of the National Collection of Industrial and Marine Bacteria at the Torry Research Station, in Aberdeen, Scotland, on 12th December, 1983 and has received the accession number NCIB 11936.

The size of the plasmid pPZ12 is, however, relatively small at 6.2 kb, which leads to a rather large number of colonies of host cells being selected in step (e) of the invention which do not in fact possess reduced restriction activity. The number of such colonies can be reduced, either by reducing the total number of transformations effected in step (c), e.g. by reducing the protoplast concentration and/or the plasmid DNA concentration of the mixture, or by increasing the size of the plasmid DNA, e.g. to about 10 to 15 kb, by adding to it, by well-known genetic engineering techniques, further DNA from a Streptomycete or any other source.

Another particular plasmid, which can be used in the process of the invention without further addition of DNA, is the hybrid cosmid pPZ74 of size 12.5 kb, the preparation of which from the plasmid pPZ12 is described in our patent application (Ref. PLC 386) filed concurrently with this application, the contents of which are incorporated herein by reference.

The particular host cells which have been used in the Examples of the present invention which follow are those of an oxytetracycline-sensitive strain of Streptomyces rimosus, designated 4018S, which has been deposited for purposes of patent procedures under the Budapest Treaty in the same Culture Collection on the same date, and received the accession number NCIB 11937.

The following are Examples of the process of the invention.

- 16 -

EXAMPLE I

(a) Spores of <u>Streptomyces rimosus</u> M4018S are treated with N-Methyl-N'-nitrosoguanidine, as described by Delic <u>et al</u> in Mutation Research, <u>9</u>(1970), 167-182.

(b) Spores which have survived the mutagenic treatment ($10^6$-$10^7$ spores) are inoculated into 30ml of BBL Trypticase Soy broth (Becton Dickinson & Co. Product No. 11768) with $MgCl_2.6H_2O$ (1g/1) and glycine (5g/1), contained in a 350ml Erlenmeyer flask. The flask of supplemented broth and mutagenised spores is incubated on a rotary shaker at 28°C for 44 hours at 250r.p.m. The mycelium is then harvested by centrifugation, washed by resuspending in a 10.3% aqueous sucrose solution and centrifuged again. The washing procedure with 10.3% sucrose is repeated. The washed mycelium (c. 0.3-0.5g wet weight) is suspended in 5ml of lysozyme solution of the following composition

| | |
|---|---|
| 10.3% sucrose | 100ml |
| 0.25M TES buffer, pH 7.2 (1) | 10ml |
| 2.5% $K_2SO_4$ | 2.0ml |
| 0.5% $KH_2PO_4$ | 1.0ml |
| 20.3% $MgCl_2.6H_2O$ | 0.25ml |
| 14.7% $CaCl_2.2H_2O$ | 0.25ml |
| Trace elements solution (2) | 0.2ml |
| Lysozyme Grade I (3) | 100mg |

(1) Sigma Chemical Co. Ltd. Product No. T.1375

(2) Okanishi <u>et al</u>, J. Gen. Microbiol. 1974, <u>80</u>, 389-400

(3) Sigma Chemical Co. Ltd. Product No. L.6876 Incubation of the mycelium in lysozyme solution is performed at 32°C and is continued for up to 6 hours or until microscopic examination reveals an abundance of protoplasts and very few hyphal fragments. At this stage the preparation if filtered through non-absorbent cotton wool to remove any remaining large hyphal fragments and the protoplasts are recovered by centrifugation of the filtrate. The protoplasts are washed by resuspension in medium 'P' (Okanishi et al, loc. cit). After centrifugation, the washed protoplasts are resuspended in medium 'P' and distributed to 2ml ampoules in 0.1ml aliquots. Ampoules are stored at -20°C until required and each should contain about $10^9$ protoplasts.

(c) Plasmid pPZ12 is isolated from Streptomyces albus AT95 using the method described by Chater et al, Current Topics in Microbiology and Immunology, 1982, 96, 69-95. The isolated plasmid DNA is finally dissolved in water to produce a concentration of 1µg of pPZ12 per 5µl of water. This plasmid preparation is used to transform the protoplasts produced from mutagenised spores of Streptomyces rimosus M4018S by adding 5-10µl to one ampoule of protoplasts from (b) thawed rapidly at 30°C in a water bath. After addition of the plasmid to the protoplasts, 0.5ml of a transformation medium is added within 20 seconds and mixed thoroughly by withdrawing the solution into a 1ml pipette and expelling it several times in quick succession. The transformation medium used is made from the following ingredients autoclaved separately:-

| | |
|---|---|
| 10.3% sucrose | 25 ml |
| 14.7% $CaCl_2.2H_2O$ | 10 ml |
| 0.5 M Tris/Maleic acid buffer pH 8.0 | 10 ml |
| 2.5% $K_2SO_4$ | 2 ml |
| Trace elements solution (1) | 0.05 ml |
| Distilled water | 28 ml |

(1)  Okanishi et al (loc. cit)

3 ml of the above solution are then added to 1 g of polyethylene glycol 1000 to constitute the transformation medium.

(d)  The fused protoplast mixture is diluted at least ten-fold in medium 'P' to reduce the concentration of polyethylene glycol.  Aliquots of the diluted protoplast fusion mixture are then spread on Petri dishes containing protoplast regeneration medium R9.  Medium R9 is prepared as two separate solutions R8(A) and R9(B) which are mixed in equal volumes after sterilization.

Solution R9(A) is prepared by dissolving 11.5 g TES buffer in 500 ml demineralised water, adjusting the pH to 7.4, adding 10 g yeast extract and stirring until dissolved, adding 427 g sucrose and stirring until dissolved, making up the volume to 1 litre with demineralised water and dispensing 100 ml aliquots into 350 ml flasks.

Solution R9(B) has the following composition:-

| | |
|---|---|
| Glucose | 20.0 g |
| $K_2SO_4$ | 0.5 g |
| $MgCl_2.6H_2O$ | 8.1 g |
| $NaNO_3$ | 4.0 g |
| KCl | 1.0 g |
| $MgSO_4.7H_2O$ | 0.4 g |
| $CaCl_2.2H_2O$ | 14.7 g |
| Casamino acids (Difco) | 0.8 g |
| $FeSO_4.7H_2O$ (1% solution) | 2.0 ml |
| Trace elements solution (1) | 4.0 ml |
| Demineralised water | to 1 litre |

100 ml aliquots of this solution are dispensed to 350 ml flasks containing 4.4 g Difco agar per flask.

After sterilisation, one flask of R9(A) is mixed with one flask of R9(B) and 1 ml of 1% aqueous $KH_2PO_4$ is added. The mixture is cooled to 60°C and poured into Petri dishes. The plates of poured medium are dried before use to reduce the weight of the medium by 5%.

(e) After incubation for 28 hours at 30°C, plates of regenerating protoplasts are overlayered with R9 medium lacking agar but containing thiostrepton at a concentration of 200 μg per ml. This procedure selects for regenerated protoplasts transformed with pPZ12 and visible colonies 1-2 mm in diameter are produced after incubation of the regeneration plates for a further 6 to 10 days. Each of the transformed colonies is purified by streaking onto Emerson's agar containing thiostrepton at 50μg/ml of medium.

(f) The purified colonies are grown separately on Emerson's agar in the absence of thiostrepton (to encourage plasmid loss) and progeny from each original transformed colony are tested in order to detect a variant lacking pPZ12. (Thiostrepton-sensitive variants are presumed to have lost pPZ12.) This is done by replica-plating the progeny of transformed colonies to Emerson's agar medium containing thiostrepton at 50 μg/ml of medium. When a thiostrepton-sensitive variant has been isolated from each original transformant, protoplasts are prepared as described in (b) above and transformed again with pPZ12 isolated from Streptomyces albus AT95 and regenerated as in (c) and (d) above. A mutant possessing reduced restriction activity is indicated by a considerably enhanced transformation frequency, the efficiency of transformation of the mutant with pPZ12 being up to $10^6$ times greater, when compared with transformations of the original, unmutated M4018S parent.

## EXAMPLE II

The procedure of Example I is repeated, but using in step (c) the plasmid pPZ74, the preparation of which is described in our patent application (Ref. PLC 387) filed concurrently herewith.

CLAIMS

1.    A method for obtaining Streptomycete host cells having reduced restriction activity, which comprises the steps of:

(a)   mutagenising a strain of a Streptomycete species to provide host cells of differing genetic characteristics;

(b)   growing the surviving host cells and forming protoplasts thereof;

(c)   transforming the protoplasts with plasmid DNA from another source, such plasmid DNA containing an origin of replication and a selective marker for the Streptomycete host, as well as any DNA sequences necessary for replication and selective culture from the other source;

(d)   regenerating host cells from the transformed protoplasts containing the plasmid DNA;

(e)   selecting those host cells which have received the plasmid DNA, and been unable to restrict it, by selecting for expression of the selective marker;

(f)   confirming that the host cells have reduced restriction activity; and

(g)   if desired, repeating steps (a) to (f) to reduce further the restriction activity of the host cells and confirm their further reduced restriction activity.

2.    A method according to claim 1, wherein in step (f) the selected host cells from step (e) are further selected for loss of the plasmid DNA from another source introduced in step (c) and tested for reduced restriction activity, thereby providing host cells of reduced restriction activity without the selective marker.

- 22 -

3.   A method according to claim 1 or claim 2, wherein in step (a) mutagenesis is accelerated e.g. by treatment with a chemical mutagen.

4.   A method according to claim 3, in which said mutagen is N-methyl-N'-nitrosoguamidine.

5.   A method according to any preceding claim, wherein in step (c) transformation is effected by mixing a buffered aqueous suspension of said plasmid DNA with said protoplasts and adding an agent which promotes transfer of plasmid DNA into the protoplasts.

6.   A method according to claim 5, in which said agent is a 20-25% (g/100 ml) aqueous solution of polyethylene glycol of molecular weight 1000 to 6000.

7.   A method according to claim 5 or claim 6, in which the number of transformations effected is reduced by reducing the protoplast concentration and/or the plasmid DNA concentration in the mixture.

8.   A method according to any preceding claim, wherein in steps (c) and (e) the selective marker is one for resistance to an antibiotic and those host cells which continue to grow when exposed to the antibiotic are selected.

9.   A method according to any preceding claim, wherein in step (c) the plasmid DNA is derived from a plasmid from another Streptomycete strain, by addition of a selective marker for resistance to an antibiotic.

10.   A method according to claim 9, in which said plasmid DNA is derived from plasmid pIJ 101, by addition of a thiostrepton-resistant marker followed by in vivo deletion of DNA incompatible with Streptomyces rimosus.

11. A method according to claim 10, in which said plasmid DNA is that of the hybrid cosmid pPZ 74.

12. A method according to any of claims 1 to 8, wherein in step (c) the plasmid DNA is derived from a microorganism other than a streptomycete, by addition of a fragment of Streptomycete DNA containing an origin of replication for streptomycetes and a selective marker for resistance to an antibiotic.

13. A method according to any preceding claim, wherein in step (a) the host cells are those of <u>Streptomyces</u> <u>rimosus</u> 4018S (NCIB 11937).

14. Streptomyces host cells having reduced restriction activity, obtained by a method according to any preceding claim.